(19) Europäisches Patentamt
European Patent Office
Office européen des brevets

(11) EP 4 101 450 A1

(12) EUROPEAN PATENT APPLICATION
published in accordance with Art. 153(4) EPC

(43) Date of publication:
14.12.2022 Bulletin 2022/50

(21) Application number: 20917611.4

(22) Date of filing: 15.07.2020

(51) International Patent Classification (IPC):
*A61K 31/427* *(2006.01)* *A61P 11/00* *(2006.01)*
*A61P 31/14* *(2006.01)*

(86) International application number:
PCT/CN2020/102142

(87) International publication number:
WO 2021/155654 (12.08.2021 Gazette 2021/32)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 03.02.2020 CN 202010078807

(71) Applicant: Academy of Military Medical Sciences
Beijing 100850 (CN)

(72) Inventors:
- ZHONG, Wu
Beijing 100850 (CN)
- HU, Zhihong
Beijing 100850 (CN)
- XIAO, Gengfu
Beijing 100850 (CN)
- CAO, Ruiyuan
Beijing 100850 (CN)
- WANG, Manli
Beijing 100850 (CN)
- ZHANG, Leike
Beijing 100850 (CN)
- LI, Wei
Beijing 100850 (CN)
- FAN, Shiyong
Beijing 100850 (CN)
- LI, Song
Beijing 100850 (CN)

(74) Representative: Weickmann & Weickmann PartmbB
Postfach 860 820
81635 München (DE)

Remarks:
The complete document including Reference Table(s) and the Sequence Listing(s) can be downloaded from the EPO website

# (54) APPLICATION OF RITONAVIR IN TREATING SARS-COV-2 INFECTION

(57) A use of ritonavir, or a geometric isomer, pharmaceutically acceptable salt, solvate, and/or hydrate thereof, and a pharmaceutical composition containing the above compound in treating a SARS-CoV-2 infections.

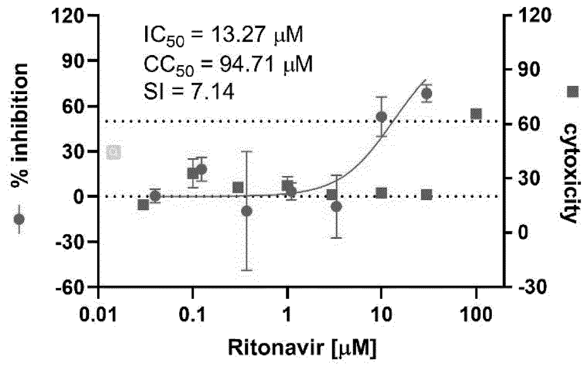


Figure 1

EP 4 101 450 A1

## Description

The invention is based on and claims the benefit of priority from Chinese application No. 202010078807.2, filed on February 3, 2020, the disclosure of which are incorporated herein by reference in its entirety.

## Technical Field

The present application relates to use of ritonavir represented by Formula I, a geometric isomer, a pharmaceutically acceptable salt and/or a solvate and/or a hydrate thereof, and a pharmaceutical composition comprising the above-mentioned compound in the treatment of a SARS-CoV-2 infection.

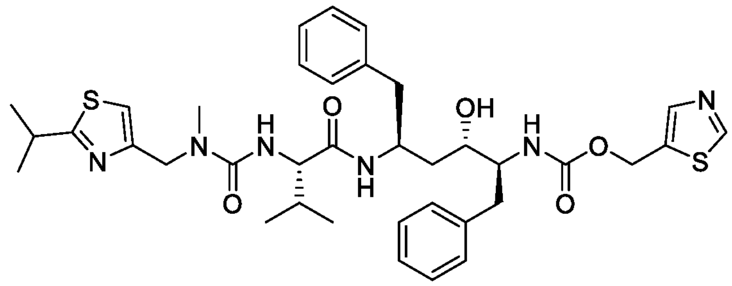

I

## Background Art

The compound represented by Formula I, known as ritonavir in English, has a chemical name of [5S-(5R,8R,10R,11R)] -10-hydroxy-2-methyl-5-(1-methylethyl)-1-[2-(1-methylethyl)- 4-thiazolyl]-3,6-dioxo-8,11-bis(phenylmethyl)-2,4,7,12-tetraazatridecan-13-oic acid, 5-thiazolyl methyl ester, it is an HIV protease inhibitor, and approved on June 29, 1999 for the treatment of HIV infection.

Ritonavir is a peptide inhibitor of HIV-1 and HIV-2 proteases. It inhibits the HIV proteinase, which renders the enzyme incapable of regulating the Gag-Pol polyprotein precursor, resulting in noninfectious immature HIV particles. The *in vitro* antiviral activity of ritonavir was studied by means of acutely infected lymphoblastoid cells and peripheral blood lymphocytes.

Depending upon the HIV-1 isolates and the cells employed, the concentration of ritonavir that inhibits 50% (EC50) of viral replication is at the range of 3.8 to 153 nM. The average $EC_{50}$ for low passage clinical isolates was 22 $nmol \cdot L^{-1}$ (n=13). Studies has reported that during SARS in 2003, after patients were administrated 400mg of lopinavir and 100mg of ritonavir, the adverse clinical outcome (ARDS or death) was significantly lower in the treatment group than in the control group (2.4% vs 28.8%, p,0.001).

However, researchers believe that lopinavir is the substance that inhibits SARS-CoV, while ritonavir does not have inhibitory activity against SARS-CoV. The role of ritonavir is to inhibit the CYP3A mediated metabolism of lopinavir and thereby potentiates the serum level of lopinavir (Thorax 2004; 59: 252 - 256). Further literature reported that ritonavir was ineffective against SARS-CoV (Biochemical and Biophysical Research Communications 318 (2004) 719-725).

The 2019 novel coronavirus (2019-nCoV) is a new coronavirus strain that has never been found in humans before. On February 11, 2020, the International Committee on Taxonomy Viruses (ICTV) announced that the official name of 2019 novel Coronavirus (2019-nCoV) is called severe acute respiratory syndrome coronavirus 2 (SARS-CoV-2). On the same day, the World Health Organization (WHO) announced that the official name of the disease caused by this virus is COVID-19. The symptoms of SARS-CoV-2 virus infection are mainly pneumonia, and can be divided into simple infection, mild pneumonia, severe pneumonia, acute respiratory distress syndrome, sepsis, septic shock and so on according to the severity of disease. Patients with simple infection may have non-specific symptoms, such as fever, cough, sore throat, nasal congestion, fatigue, headache, muscle pain or discomfort, and the elderly and immunosuppressed people may have atypical symptoms. Patients with mild pneumonia mainly have cough, dyspnea and polypnea. Severe pneumonia can be seen in adolescents, adults or children, and the main symptoms of which include increased breathing frequency, severe respiratory failure or dyspnea, central cyanosis, drowsiness, unconsciousness or convulsion, gasp, etc. The lung images of acute respiratory distress syndrome are bilateral ground glass shadows, which cannot be completely explained by effusion, lobar exudation or atelectasis or lung mass shadows, and the main symptom of which is pulmonary edema. Patients with sepsis often have fatal organ dysfunction, and the most critical patients are those with septic shock, and they may have a high probability of death.

At present, the SARS-CoV-2 infection is mainly treated with supportive therapy in clinic, and no specific antiviral drug is available.

**Contents of the Invention**

The purpose of the present application is to discover a drug with an antiviral activity against SARS-CoV-2, which can be used for the treatment of a relative disease caused by SARS-CoV-2 infection, for example, simple infection (such as fever, cough and sore throat), pneumonia, acute respiratory infection or severe acute respiratory infection, hypoxic respiratory failure and acute respiratory distress syndrome, sepsis and septic shock, etc.

Through creative research in the present application, it is found in the *in vitro* experiments of SARS-CoV-2 infected cells that ritonavir represented by Formular I has a function of inhibiting the replication of SARS-CoV-2, and a good potential therapeutic effect in the treatment of a disease caused by a SARS-CoV-2.

The present application relates to a compound represented by Formula I, a geometric isomer, a pharmaceutically acceptable salt and/or a solvate or a hydrate thereof:

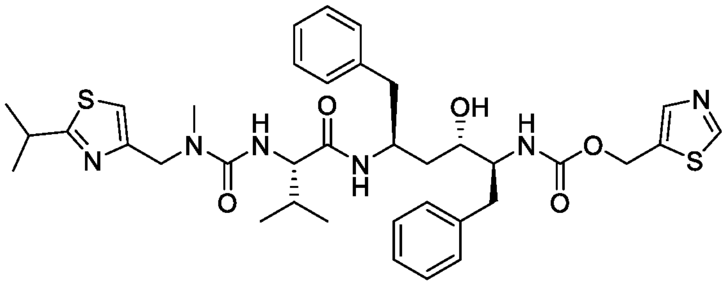

I.

According to the present application, the pharmaceutically acceptable salts of the compound represented by Formula I of the present application include an inorganic or organic acid salt thereof and an inorganic or organic base salt thereof. The present application relates to all forms of the above salts, including but not limited to: sodium salt, potassium salt, calcium salt, lithium salt, meglumine salt, hydrochloride salt, hydrobromide salt, hydroiodide salt, nitrate salt, sulfate, hydrogen sulfate, phosphate, hydrogen phosphate, acetate, propionate, butyrate, oxalate, pivalate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and embonate and so on.

According to the present application, the compound represented by Formula I can inhibit the replication of SARS-CoV-2 in a cell and reduce the nucleic acid load of SARS-CoV-2 in a cell culture.

After creative invention and research, the inventors of the present application have discovered some new features of the compound represented by Formula I: the compound ritonavir represented by Formula I can reduce the viral nucleic acid load in SARS-CoV-2 infected cells at micromolar concentration level.

The present application also relates to use of ritonavir represented by Formula I, a geometric isomer, a pharmaceutically acceptable salt and/or a solvate and/or a hydrate thereof in the manufacture of a medicament for the prevention and/or the treatment of a disease or an infection caused by a SARS-CoV-2 (e.g., a respiratory disease (including but not limited to simple infection (such as fever, cough and sore throat), pneumonia, acute or severe acute respiratory infection, hypoxic respiratory failure and acute respiratory distress syndrome), sepsis and septic shock, etc.),

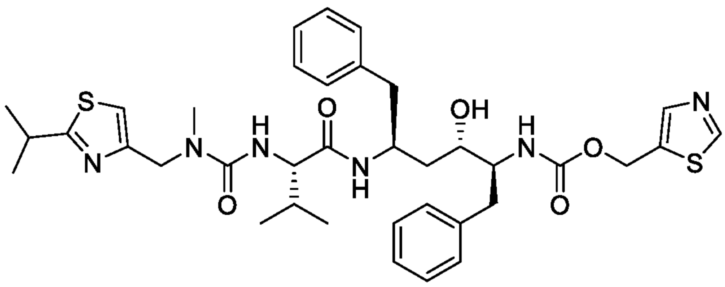

I.

The present application also relates to use of ritonavir represented by Formula I, a geometric isomer, a pharmaceutically acceptable salt and/or a solvate and/or a hydrate thereof in the manufacture of a medicament as a SARS-CoV-2 inhibitor, or in the manufacture of a medicament for inhibiting the replication or reproduction of SARS-CoV-2 in a cell (e.g., a cell of mammal),

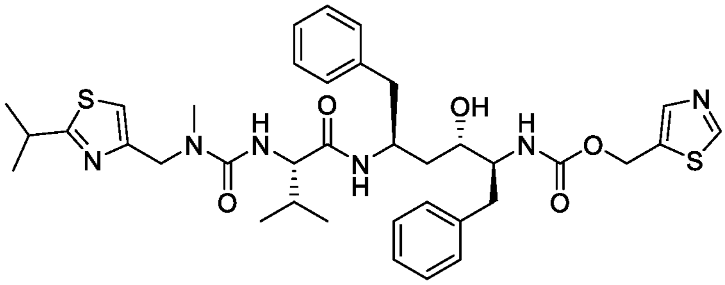

I.

The present application also relates to use of a pharmaceutical composition in the manufacture of a medicament as a SARS-CoV-2 inhibitor, or in the manufacture of a medicament for inhibiting the replication or reproduction of SARS-CoV-2 in a cell (e.g., a cell of mammal), wherein the pharmaceutical composition comprises ritonavir represented by Formula I, a geometric isomer, a pharmaceutically acceptable salt and/or a solvate and/or a hydrate thereof,

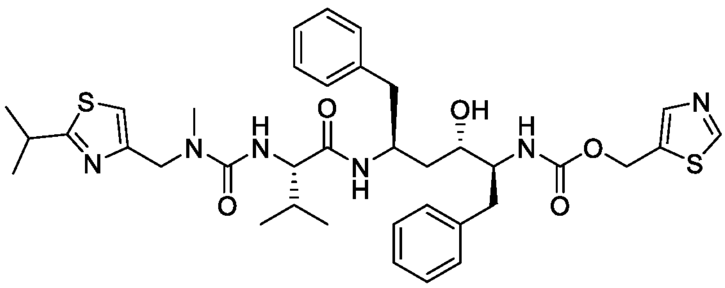

I

preferably, the pharmaceutical composition further comprises a pharmaceutically acceptable carrier or excipient; specifically, the pharmaceutical composition is a solid preparation, an injection, an external preparation, a spray, a liquid preparation, or a compound preparation.

The present application also relates to use of a pharmaceutical composition in the manufacture of a medicament for the prevention and/or the treatment of a disease or an infection caused by a SARS-CoV-2 (e.g., a respiratory disease (including but not limited to simple infection (such as fever, cough and sore throat), pneumonia, acute or severe acute respiratory infection, hypoxic respiratory failure and acute respiratory distress syndrome), sepsis and septic shock, etc.), wherein the pharmaceutical composition comprises ritonavir represented by Formula I, a geometric isomer, a pharmaceutically acceptable salt and/or a solvate and/or a hydrate thereof,

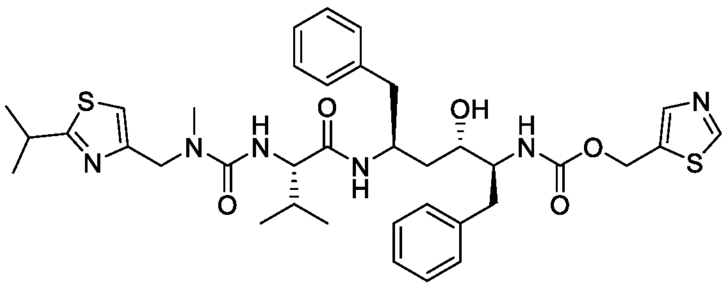

I

preferably, the pharmaceutical composition further comprises a pharmaceutically acceptable carrier or excipient; specifically, the pharmaceutical composition is a solid preparation, an injection, an external preparation, a spray, a liquid

preparation, or a compound preparation.

The present application also relates to a method for preventing and/or treating a disease or a virus infection in a mammal in need thereof or a method for inhibiting the replication or reproduction of SARS-CoV-2 in a mammal in need thereof, wherein the method comprises administering to the mammal in need thereof a prophylactically and/or therapeutically effective amount of ritonavir represented by Formula I, a geometric isomer, a pharmaceutically acceptable salt and/or a solvate and/or a hydrates thereof or a prophylactically and/or therapeutically effective amount of pharmaceutical composition comprising a compound represented by Formula I, a geometric isomer, a pharmaceutically acceptable salt and/or a solvate and/or a hydrate thereof,

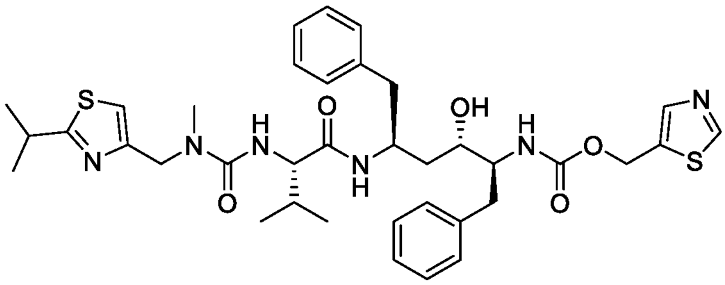

I

wherein the disease includes a disease caused by a SARS-CoV-2 (e.g., a respiratory disease (including but not limited to simple infection (such as fever, cough and sore throat), pneumonia, acute or severe acute respiratory infection (SARI), hypoxic respiratory failure and acute respiratory distress syndrome), sepsis and septic shock, etc.), the virus infection includes an infection caused by a SARS-CoV-2.

The present application also relates to ritonavir represented by Formula I, a geometric isomer, a pharmaceutically acceptable salt and/or a solvate and/or a hydrate thereof or a pharmaceutical composition comprising ritonavir represented by Formula I, a geometric isomer, a pharmaceutically acceptable salt and/or a solvate and/or a hydrate thereof, for use as a SARS-CoV-2 inhibitor, or for use in inhibiting the replication or reproduction of SARS-CoV-2 in a cell (e.g., a cell of mammal),

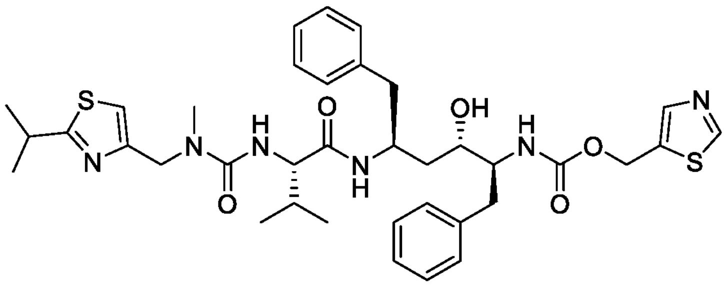

I

preferably, the pharmaceutical composition further comprises a pharmaceutically acceptable carrier or excipient.

The present application also relates to ritonavir represented by Formula I, a geometric isomer, a pharmaceutically acceptable salt and/or a solvate and/or a hydrate thereof or a pharmaceutical composition comprising ritonavir represented by Formula I, a geometric isomer, a pharmaceutically acceptable salt and/or a solvate and/or a hydrate thereof, for use in preventing and/or treating a disease or an infection caused by a SARS-CoV-2 (e.g., a respiratory disease (including but not limited to simple infection (such as fever, cough and sore throat), pneumonia, acute respiratory infection or severe acute respiratory infection, hypoxic respiratory failure and acute respiratory distress syndrome), sepsis and septic shock, etc.),

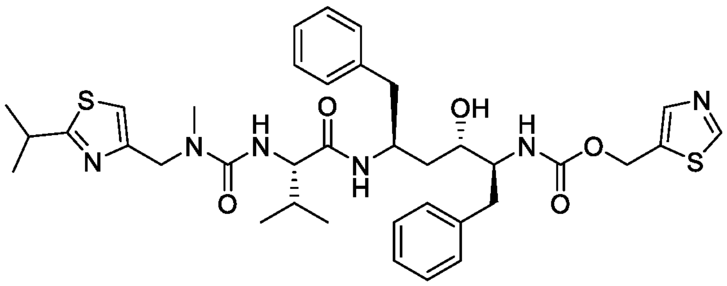

I

preferably, the pharmaceutical composition further comprises a pharmaceutically acceptable carrier or excipient.

In some embodiments, the disease caused by a SARS-CoV-2 described in the present application is COVID-19.

In the present application, the official name of the term "2019 novel coronavirus (2019-nCoV)" is severe acute respiratory syndrome coronavirus 2 (SARS-CoV-2).

In the present application, the official name of the term "disease caused by 2019 novel coronavirus (2019-nCoV)" is COVID-19.

In some embodiments, the mammal includes bovine, equine, caprid, suidae, canine, feline, rodent, primate, wherein the preferred mammal is a human, a cat, a dog, or a pig.

The pharmaceutical composition described in the present application can be prepared into various forms according to different administration routes.

According to the present application, the pharmaceutical composition can be administered in any one of the following routes: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration with the help of an explant reservoir. Among them, oral, intraperitoneal or intravenous administration is preferred.

When orally administered, the compound represented by Formular I, a geometric isomer, a pharmaceutically acceptable salt and/or a solvate and/or a hydrate thereof can be prepared into any form of orally acceptable preparation, including but not limited to a tablet, a capsule, an aqueous solution or an aqueous suspension. Generally, the carrier for use in a tablet includes lactose and corn starch, and a lubricant such as magnesium stearate can also be added. The diluent for use in a capsule generally includes lactose and dry corn starch. The aqueous suspension is usually used by mixing an active ingredient with a suitable emulsifier and a suitable suspending agent. If necessary, a sweetener, a flavoring agent or a coloring agent can also be added to the above-mentioned forms of oral preparation.

When rectally administered, the compound represented by Formular I, a geometric isomer, a pharmaceutically acceptable salt and/or a solvate, and/or a hydrate thereof can generally be prepared in a form of suppository, which is prepared by mixing the drug with a suitable nonirritating excipient. The excipient is present in solid state at room temperature, but melts at the rectal temperature to release the drug. Such excipient includes cocoa butter, beeswax and polyethylene glycol.

When topically administered, especially for the treatment of easily accessible affected-surface or organ, such as eye, skin, or lower intestinal neurological disease by topical application, the compound represented by Formular I, a geometric isomer, a pharmaceutically acceptable salt and/or a solvate and/or a hydrate thereof can be prepared in various forms of topical preparations according to different affected-surfaces or organs, the specific instructions are as follows:

When topically administered to eye, the compound represented by Formular I, a geometric isomer, a pharmaceutically acceptable salt and/or a solvate and/or a hydrate thereof can be formulated into a preparation form such as micronized suspension or solution, the carrier used is isotonic sterile saline with a certain pH, and a preservative such as benzyl chloride alkoxide may or may not be added. In addition, for administration to eye, the compound can also be prepared in a form of ointment such as vaseline ointment.

When topically administered to skin, the compound represented by Formular I, a geometric isomer, a pharmaceutically acceptable salts and/or a solvate and/or a hydrate thereof can be prepared into a suitable form such as an ointment, a lotion or a cream, in which the active ingredient is suspended or dissolved in one or more carriers. The carrier for use in an ointment includes, but is not limited to mineral oil, liquid petrolatum, white petrolatum, propylene glycol, polyethylene oxide, polypropylene oxide, emulsifying wax, and water. The carrier for use in a lotion or a cream includes, but is not limited to mineral oil, sorbitan monostearate, Tween-60, cetyl ester wax, hexadecenyl aryl alcohol, 2-octyldodecanol, benzyl alcohol and water.

When topically administered to lower intestinal tract, the compound represented by Formular I, a geometric

isomer, a pharmaceutically acceptable salt and/or a solvate and/or a hydrate thereof can be prepared into a form such as rectal suppository as described above or a suitable enema preparation form, in addition, a topical transdermal patch can also be used.

The compound represented by Formular I, a geometric isomer, a pharmaceutically acceptable salt and/or a solvate and/or a hydrate thereof can also be administered in a preparation form of sterile injection, including sterile injectable aqueous solution or oil suspension, or sterile injectable solutions, wherein the usable carrier and solvent includes water, Ringer's solution and isotonic sodium chloride solution. In addition, a sterilized non-volatile oil such as monoglyceride or diglyceride can also be used as solvent or suspension media.

The drugs of the above various preparation forms can be prepared according to conventional methods in the pharmaceutical field.

In the present application, the term "therapeutically effective amount" or "prophylactically effective amount" refers to an amount that is sufficient to treat or prevent a patient's disease but is sufficiently low to avoid serious side effects (at a reasonable benefit/risk ratio) within a reasonable medical judgment. The prophylactically or therapeutically effective amount of the compound will change according to the factors such as the selected specific compound (e.g., considering the efficacy, effectiveness, and half-life of compound), the selected administration route, the disease to be prevented or treated, the severity of the disease to be prevented or treated, the prevented or treated patient's age, size, weight and physical disease, medical history, duration of prevention or treatment, nature of concurrent therapy, desired prophylactic or therapeutic effect, etc., but can still be routinely determined by those skilled in the art.

In addition, it should be noted that the specific dosage and method of using the compound represented by Formular I, a geometric isomer, a pharmaceutically acceptable salt and/or a solvate and/or a hydrate thereof for different patients depends on many factors, including the patient's age, weight, gender, natural health status, nutritional status, active strength of drug, administration time, metabolic rate, severity of disease, and subjective judgment of physician. Herein it is preferred to use a dosage between 0.0001-1000 mg/kg body weight/day.

## Brief Description of the Drawings

Figure 1 shows that ritonavir can effectively reduce the viral nucleic acid load in Vero E6 cells infected by SARS-CoV-2. Ritonavir can reduce the viral RNA load in the cells 48 hours after the cells were infected by SARS-CoV-2, and the inhibitory activity is dose-dependent. In Figure 1, the left ordinate is the copy number of viral RNA in the sample, the right ordinate is the cytoxicity of the drug, and the abscissa is the drug concentration.

## Specific Models for Carrying Out the Invention

The following examples are illustrative preferred embodiments of the present application and do not constitute any limitation to the present application.

Example 1: Experiment on ritonavir reducing viral nucleic acid load in SARS-CoV-2 infected cell s

(1) Drug treatment of virus-infected cells

Vero E6 cells (purchased from ATCC, Catalog No. 1586) was inoculated on a 24-well plate, cultured for 24 hours; then virus infection was carried out. Specifically, SARS-CoV-2 (2019-nCoV) virus (nCoV-2019BetaCoV/Wuhan/WIV04/2019 strain, provided by Wuhan Institute of Virology, Chinese Academy of Sciences) was diluted with 2% cell maintenance solution (formulation: FBS (purchased from Gibco company, Catalog No. 16000044) was added to MEM (purchased from Gibco, Catalog No. 10370021) at a volume ratio of 2%, thereby obtaining the 2% cell maintenance solution) to a corresponding concentration, and then added to a 24-well plate so that each well contained a viral load of $100TCID_{50}$. Ritonavir (purchased from Selleck Chemicals, Catalog No. S1185) was diluted with 2% cell maintenance solution to corresponding concentrations and added separately to the corresponding wells, so that the final concentrations of the drugs were 100 μM, 33 μM, 11 μM, 3.7 μM, 1.23μM, 0.41μM, 0.14μM, respectively, then the plate was placed in a 37°C, 5% $CO_2$ incubator and cultured for 48 hours. To the vehicle control group, the 2% cell maintenance solution without any test drugs was added.

(2) RNA extraction

RNA extraction kit was purchased from Qiagen Company, Catalog No. 74106. The consumables (spin columns, RNase-free 2ml collection tubes, etc.) and reagents (RLT, RW1, RPE, RNase-free water, etc.) involved in the following RNA extraction steps were part of the kit. The following extraction steps were recommended steps in the kit instruction.

1) 100μL of the supernatant was taken from the tested plate and added to a nuclease-free EP tube, then 350μL of Buffer RLT was added to each well and mixed by beating with a transfer liquid gun until complete lysis was achieved, then centrifugation was carried out to obtain a supernatant;

2) an equal volume of 70% ethanol was added to the supernatant obtained in 1) and mixed well;

3) the mixture solution obtained in 2) was transferred to a RNase-free spin column, and centrifuged at 12000 rpm for 15 seconds, and the waste liquid was discarded;

4) 700μL of Buffer RW1 was added to the spin column, and centrifuged at 12000 rpm for 15 seconds to clean the spin column, and the waste liquid was discarded;

5) 500μL of Buffer RPE was added to the spin column, and centrifuged at 12000 rpm for 15 seconds to clean the spin column, and the waste liquid was discarded;

6) 500μL of Buffer RPE was added to the spin column, and centrifuged at 12000 rpm for 2min to clean the spin column, the waste was discarded;

7) a new RNase-free 2ml collection tube was used for replacement, centrifugation was carried out at 12000 rpm for 1 min, the spin column was dried, and then the spin column was transferred to a 1.5ml collection tube in step 8);

8) a new 1.5ml collection tube was used for replacement, in which the spin column dried in step 7) was placed, and 30μl of RNase-free water was added to the spin column, and centrifugation was carried out at 12000 rpm for 2 minutes, the obtained eluate contained the corresponding RNA, then the RNase inhibitor (purchased from NEB company, Catalog No. M0314L) was added, and Nano Drop (purchased from Thermo scientific, Nano Drop One) was used to detect each RNA concentration.

(3) RNA reverse transcription

In the experiment, the reverse transcription kit (PrimeScript™ RT reagent Kit with gDNA Eraser, Catalog No. RR047Q) produced by TaKaRa was used for RNA reverse transcription. The steps were as follows.

①Removal of gDNA: RNA samples of each experimental group were collected, 1 μg of each sample was taken for reverse transcription. First, 2μl of 5× gDNA Eraser Buffer was added to the RNA sample of each experimental group, the reaction system was supplemented with RNase-free water to reach 10μl, mixed well, and subjected to water bath at 42°C for 2 min to remove the gDNA that might be present in the sample;

②Reverse transcription: Appropriate amounts of enzyme, primer Mix and reaction buffer were added to the sample obtained in ① RNase-free water was added to supplement to reach a volume of 20 μl, the reaction was performed in a water bath at 37°C for 15 minutes, and then in water bath at 85°C for 5 seconds, to obtain cDNA by transcription.

(4) Real-time PCR

Fluorescence quantitative PCR was used to detect the number of copies per milliliter of the original virus solution.

The reaction system was mixed by using TB Green Premix (Takara, Cat#RR820A), and the amplification reaction and reading were carried out with StepOne Plus Real-time PCR instrument (brand: ABI). The copy number contained in per milliliter of the original virus solution was calculated. The steps were as follows:

①Establishment of standard product: the plasmid pMT-RBD (the plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/ μL, $5\times10^4$ copies/μL, $5\times10^3$ copies/μL, $5\times10^2$ copies/μL, respectively. 2 μL of standard product or cDNA template was taken for qPCR reaction.

②The primer sequences used in the experiment were as follows (all indicated in the 5'-3' direction):

RBD-qF: CAATGGTTTAACAGGCACAGG

RBD-qR: CTCAAGTGTCTGTGGATCACG

③The reaction procedure was as follows:

Pre-denaturation: 95°C for 5 minutes;

Cycle parameters: 95°C for 15 seconds, 54°C for 15 seconds, 72°C for 30 seconds, a total of 40 cycles.

(5) Cytotoxicity test of drugs

The cytotoxicity test of drugs was carried out by using CCK-8 kit (Beyotime). Specific steps were as follows:

①$1\times10^4$ Vero-E6 cells (ATCC) were inoculated in a 96-well plate and cultured at 37°C for 8 hours.

②The drug was diluted with DMSO to an appropriate concentration of mother solution, and then diluted with MEM (purchased from Gibco company, Catalog No. 10370021) medium containing 2% FBS (purchased from Gibco company, Catalog No. 16000044) to the same concentration as that for the drug treatment. The original medium in the 96-well plate was discarded, 100 μL of the drug-containing MEM medium was taken and added to the cells, and three replicate wells were set for each concentration. A vehicle control (adding DMSO and medium to cells in wells, without adding drug) and a blank control (adding DMSO and medium to the wells, without cells) were set up. After the drug was added, the cells were cultrued at 37°C for 48 hours.

③20 μL of CCK-8 solution (Beyotime) was added to the well to be tested, mixed gently without generating bubbles, and cultured subsequently at 37°C for 2 hours. $OD_{450}$ was read on a microplate reader (purchased from Molecular Devices, model: SpectraMax M5), and the cell viability was calculated:

$$\text{Cell viability (\%)} = (A_{\text{(drug treatment group)}} - A_{\text{(blank control)}}) / (A_{\text{(vehicle control)}} - A_{\text{(blank control)}}) \times 100\%$$

Wherein, A was the reading of the microplate reader.

(6) Experimental results

The results of the virus proliferation inhibition experiment showed that the test compound at concentrations of 33 μM, 11.1 μM could effectively inhibit the replication of SARS-CoV-2 virus genome in the infected supernatant (See Table 1 and Figure 1).

Table 1. Antiviral test results of the test compound (ritonavir)

| Concentration (μM) | 33.33 | 11.11 | 3.70 | 1.23 | 0.41 | 0.14 | Vehicle |
|---|---|---|---|---|---|---|---|
| Copy number of virus genome | 1855399 | 2236225 | 6213595 | 5565821 | 5632446 | 6032699 | 5762396 |

The cytotoxicity test results showed that the treatment of the test compound ritonavir did not change the cell viability at all test concentrations, that was, the test compound had no toxic effect on the cells at all concentrations (See Table 2 and Figure 1).

Table 2. Cytotoxicity test results of the test compound (ritonavir)

| Concentration (μM) | 100 | 33.33 | 11.11 | 3.70 | 1.23 | 0.41 | 0.14 | Vehicle |
|---|---|---|---|---|---|---|---|---|
| Cell viability (% of vehicle control) | 44.87 | 98.84 | 97.66 | 98.80 | 92.56 | 93.95 | 84.88 | 100.24 |

**Claims**

1. Use of ritonavir represented by Formula I, a geometric isomer, a pharmaceutically acceptable salt and/or a solvate and/or a hydrate thereof in the manufacture of a medicament for the prevention and/or the treatment of a disease or an infection caused by a SARS-CoV-2 (e.g., a respiratory disease (including but not limited to simple infection (such as fever, cough and sore throat), pneumonia, acute respiratory infection or severe acute respiratory infection, hypoxic respiratory failure and acute respiratory distress syndrome), sepsis and septic shock, etc.),

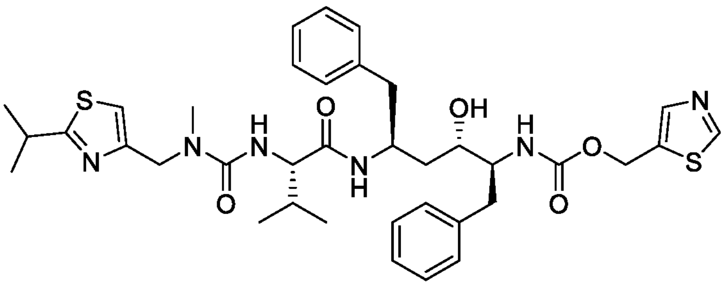

I.

2. Use of ritonavir represented by Formula I, a geometric isomer, a pharmaceutically acceptable salt and/or a solvate and/or a hydrate thereof in the manufacture of a medicament as a SARS-CoV-2 inhibitor, or in the manufacture of a medicament for inhibiting the replication or reproduction of SARS-CoV-2 in a cell (e.g., a cell of mammal),

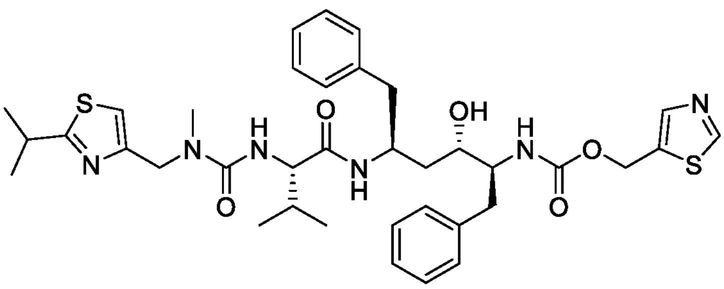

I.

3. Use of a pharmaceutical composition in the manufacture of a medicament as a SARS-CoV-2 inhibitor, or in the manufacture of a medicament for inhibiting the replication or reproduction of SARS-CoV-2 in a cell (e.g., a cell of mammal),

wherein the pharmaceutical composition comprises ritonavir represented by Formula I, a geometric isomer, a pharmaceutically acceptable salt and/or a solvate and/or a hydrate thereof,

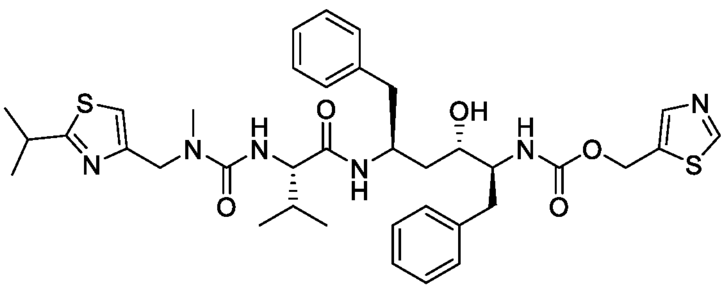

I

preferably, wherein the pharmaceutical composition further comprises a pharmaceutically acceptable carrier or excipient; specifically, the pharmaceutical composition is a solid preparation, an injection, an external preparation, a spray, a liquid preparation, or a compound preparation.

4. Use of a pharmaceutical composition in the manufacture of a medicament for the prevention and/or the treatment of a disease or an infection caused by a SAR-CoV-2 (e.g., a respiratory disease (including but not limited to simple infection (such as fever, cough and sore throat), pneumonia, acute respiratory infection or severe acute respiratory infection, hypoxic respiratory failure and acute respiratory distress syndrome), sepsis and septic shock, etc.), wherein

the pharmaceutical composition comprises ritonavir represented by Formula I, a geometric isomer, a pharmaceutically acceptable salt and/or a solvate and/or a hydrate thereof,

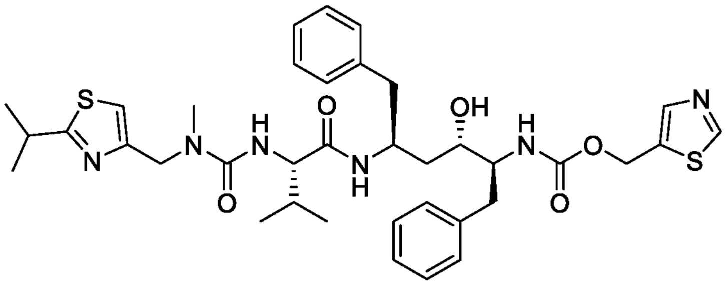

I

preferably, wherein the pharmaceutical composition further comprises a pharmaceutically acceptable carrier or excipient; specifically, the pharmaceutical composition is a solid preparation, an injection, an external preparation, a spray, a liquid preparation, or a compound preparation.

**5.** A method for preventing and/or treating a disease or a virus infection in a mammal in need thereof or a method for inhibiting the replication or reproduction of SARS-CoV-2 in a mammal in need thereof, wherein the method comprises administering to the mammal in need thereof a prophylactically and/or therapeutically effective amount of ritonavir represented by Formula I, a geometric isomer, a pharmaceutically acceptable salt and/or a solvate and/or a hydrates thereof or a prophylactically and/or therapeutically effective amount of pharmaceutical composition comprising a compound represented by Formula I, a geometric isomer, a pharmaceutically acceptable salt and/or a solvate and/or a hydrate thereof,

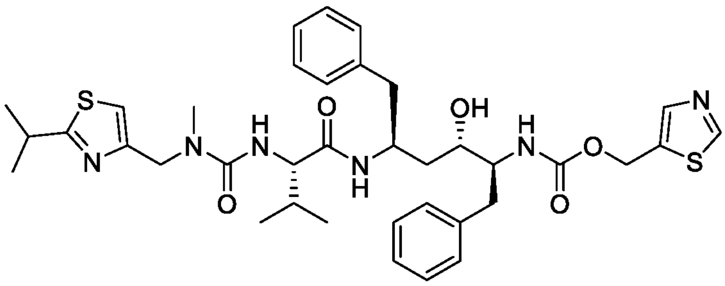

I

wherein the disease includes a disease caused by a SARS-CoV-2 (e.g., a respiratory disease (including but not limited to simple infection (such as fever, cough and sore throat), pneumonia, acute respiratory infection or severe acute respiratory infection (SARI), hypoxic respiratory failure and acute respiratory distress syndrome), sepsis and septic shock, etc.), the virus infection includes an infection caused by a SARS-CoV-2.

**6.** Ritonavir represented by Formula I, a geometric isomer, a pharmaceutically acceptable salt and/or a solvate and/or a hydrate thereof or a pharmaceutical composition comprising ritonavir represented by Formula I, a geometric isomer, a pharmaceutically acceptable salt and/or a solvate and/or a hydrate thereof, for use as a SARS-CoV-2 inhibitor, or for use in inhibiting the replication or reproduction of SARS-CoV-2 in a cell (e.g., a cell of mammal),

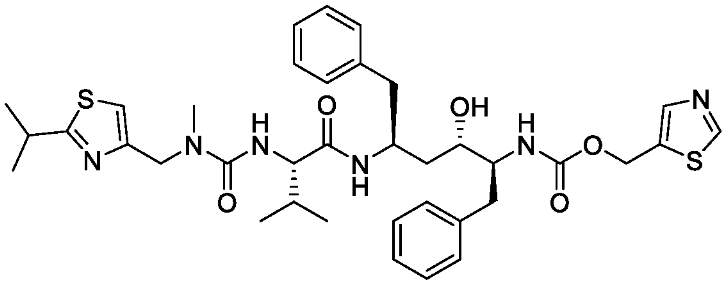

I

preferably, the pharmaceutical composition further comprises a pharmaceutically acceptable carrier or excipient.

**7.** Ritonavir represented by Formula I, a geometric isomer, a pharmaceutically acceptable salt and/or a solvate and/or a hydrate thereof or a pharmaceutical composition comprising ritonavir represented by Formula I, a geometric isomer, a pharmaceutically acceptable salt and/or a solvate and/or a hydrate thereof, for use in preventing and/or treating a disease or an infection caused by a SARS-CoV-2 (e.g., a respiratory disease (including but not limited to simple infection (such as fever, cough and sore throat), pneumonia, acute respiratory infection or severe acute respiratory infection, hypoxic respiratory failure and acute respiratory distress syndrome), sepsis and septic shock, etc.),

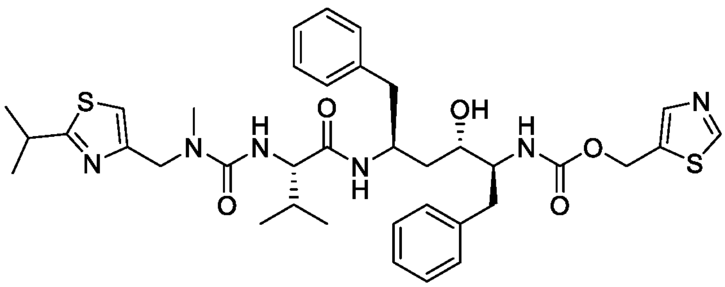

I

preferably, the pharmaceutical composition further comprises a pharmaceutically acceptable carrier or excipient.

**8.** The use according to claim 1 or 4, the method according to claim 5, or ritonavir, a geometric isomer, a pharmaceutically acceptable salt and/or a solvate and/or a hydrate thereof or the pharmaceutical composition according to claim 7, wherein the disease caused by a SARS-CoV-2 is COVID-19.

**9.** The use according to claim 2 or 3, the method according to claim 5, or ritonavir, a geometric isomer, a pharmaceutically acceptable salt and/or a solvate and/or a hydrate thereof or the pharmaceutical composition according to claim 6, wherein the mammal includes bovine, equine, caprid, suidae, canine, feline, rodent, primate, for example, is a human, a cat, a dog or a pig.

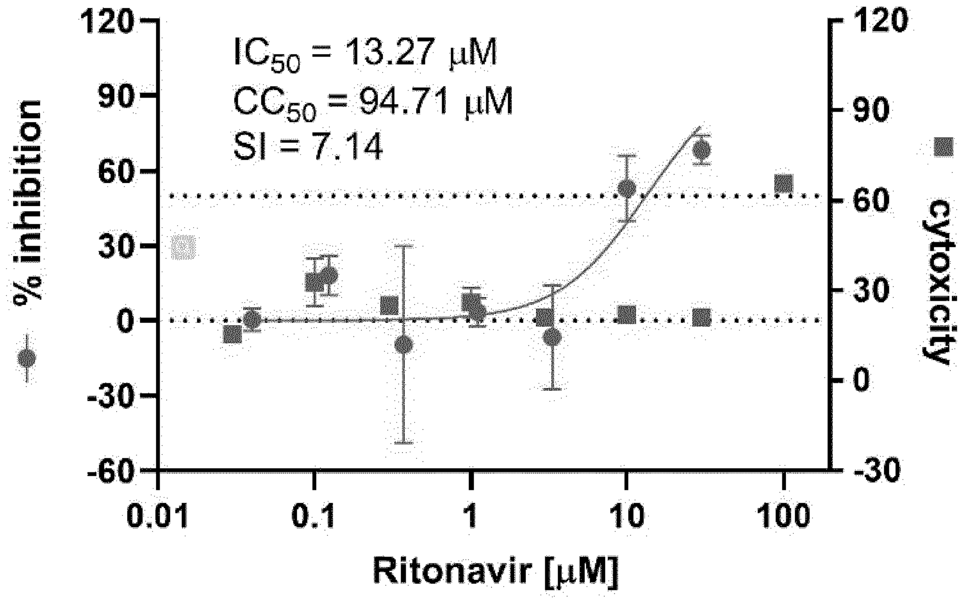
IC50 = 13.27 µM
CC50 = 94.71 µM
SI = 7.14
% inhibition
cytoxicity
120
90
60
30
0
-30
-60
120
90
60
30
0
-30
0.01
0.1
1
10
100
Ritonavir [µM]

Figure 1

## INTERNATIONAL SEARCH REPORT

International application No. **PCT/CN2020/102142**

### A. CLASSIFICATION OF SUBJECT MATTER

A61K 31/427(2006.01)i; A61P 11/00(2006.01)i; A61P 31/14(2006.01)i

According to International Patent Classification (IPC) or to both national classification and IPC

### B. FIELDS SEARCHED

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, CAPLUS(STN), CNKI, 百度学术, BAIDU XUESHU: 中国人民解放军军事科学院军事医学研究院, 钟武, 胡志红, 肖庚富, 曹瑞源, 王曼丽, 张磊砢, 李薇, 樊士勇, 李松, 利托那韦, 新冠, 新型冠状病毒, 肺炎, ritonavir, SARS-CoV-2, COVID-19, 2019-nCoV, coronavirus, pneumonia, 155213-67-5

### C. DOCUMENTS CONSIDERED TO BE RELEVANT

| Category* | Citation of document, with indication, where appropriate, of the relevant passages | Relevant to claim No. |
|---|---|---|
| PX | 凤凰网 (ifeng.com). "歌礼制药-B(01672)：利托那韦片治疗新冠肺炎的研究获浙江省相关监管部门批准 (Non-official translation: Gloria-B (01672): Ritonavir Tablets for the Treatment of COVID-19 Approved by the Relevant Regulatory Authorities of Zhejiang Province)" *https://finance.ifeng.com/c/7u87BHyOQdg,* 17 February 2020 (2020-02-17), p. 1 | 1-9 |
| PX | MAZUMDER, Muhammed Khairujjaman et al. "Suggesting Ritonavir against COVID-19/ SARS-CoV-2" *Medical Hypotheses,* Vol. 140, 30 April 2020 (2020-04-30), page 109764 | 1-9 |
| X | 中国科学院 (Bulletin of Chinese Academy of Sciences). "【湖北日报】 武汉病毒研究所等发现：3种药物对新型冠状病毒有较好抑制作用 (Non-official translation: [Hubei Daily] Wuhan Institute of Virus Research et al. Found 3 Drugs with Better Inhibitory Effect on Novel Coronavirus)" *http://www.cas.cn/zt/sszt/kjgzbd/mtbd/202001/t20200129_4732980.shtml,* 29 January 2020 (2020-01-29), page 1 | 1-9 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 October 2020** | **27 October 2020** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/102142**

**C. DOCUMENTS CONSIDERED TO BE RELEVANT**

| Category* | Citation of document, with indication, where appropriate, of the relevant passages | Relevant to claim No. |
|---|---|---|
| X | 格隆汇 (gelonghui.com). "中国医疗集团（08225.HK）旗下专案组研究发现利托那韦对新型冠状病毒有抑制作用 (Non-official translation: China Medical Group's (08225.HK) Task Force Finds Ritonavir Inhibitory Effect on Novel Coronavirus)" *https://www.gelonghui.com/news/301840,* 29 January 2020 (2020-01-29), p. 1 | 1-9 |
| X | CN 104069104 A (BINZHOU MEDICAL COLLEGE) 01 October 2014 (2014-10-01) description, paragraphs [0012] and [0013] | 6-9 |
| A | 医政管理局 (Medical Administration of National Health Commission of the People's Republic of China). "新型冠状病毒感染的肺炎诊疗方案（试行第四版）(Non-official translation: Pneumonia Treatment Protocol for Novel Coronavirus Infection (Trial Version 4))" *http://www.nhc.gov.cn/yzygj/s7653p/202001/4294563ed35b43209b31739bd0785e67.shtml,* 27 January 2020 (2020-01-27), pp. 1-10 | 1-9 |
| A | 盛月红 等 (SHENG, Yuehong et al.). "医疗队抗击新型冠状病毒肺炎疫情救援物资筹备及管理 (Non-official translation: Preparation and Management of Relief Supplies for Medical Teams Fighting the COVID-19 Outbreak)" *解放军医院管理杂志 (Hospital Administration Journal of Chinese People's Liberation Army),* Vol. 27, No. 1, 30 January 2020 (2020-01-30), pp. 4-7 | 1-9 |

Form PCT/ISA/210 (second sheet) (January 2015)

**INTERNATIONAL SEARCH REPORT**

International application No. **PCT/CN2020/102142**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **5、8-9**（部分）
because they relate to subject matter not required to be searched by this Authority, namely:

[1] Claim 5 relates to a method for preventing and/or treating diseases or viral infection in a mammal in need or for inhibiting the replication or propagation of SARS-CoV-2 in a mammal in need, and claims 8 and 9 both refer to the method described in claim 5, which relates to a method for the treatment of a disease in a human or animal body and does not comply with PCT Rule 39.1(iv).

[2] This search report is made on the basis of assuming that the subject matter of claims 5, 8 and 9 (in part) is "the use of prophylactically and/or therapeutically effective amounts of ritonavir as shown in Formula I, geometric isomers thereof or pharmaceutically acceptable salts thereof and/or solvates thereof and/or hydrates thereof or prophylactically and/or therapeutically effective amounts of compounds comprising Formula I, and pharmaceutical compositions of geometric isomers thereof or pharmaceutically acceptable salts thereof and/or solvates thereof and/or hydrates thereof in the preparation of drugs for the prevention and/or treatment of diseases or viral infection caused by SARS-CoV-2 in mammals in need or in the preparation of drugs for the inhibition of SARS-CoV-2 replication or propagation in mammals in need".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2020/102142**

| Patent document cited in search report | Publication date (day/month/year) | Patent family member(s) | Publication date (day/month/year) |
|---|---|---|---|
| CN 104069104 A | 01 October 2014 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010078807


### Non-patent literature cited in the description


- *Thorax,* 2004, vol. 59, 252-256
- *Biochemical and Biophysical Research Communications,* 2004, vol. 318, 719-725